# EUROPEAN PATENT APPLICATION

(11) **EP 3 786 964 A1**
(43) Date of publication of application: **03.03.2021**
(21) Application number: 19306039.9
(22) Date of filing: 28.08.2019
(51) Int. Cl.: G16H 10/00

(54) **METHOD AND DEVICE FOR DETERMINING REAL-TIME CONTINUOUS GLUCOSE MONITORING DATA**

(71) Applicant: Diabnext SAS, 78000 Versailles (FR)
(72) Inventor: NICOLAS, Laurent, 75008 Paris (FR)
(74) Representative: August Debouzy

(57) **Abstract**

It is proposed a computer-implemented method for determining real-time continuous glucose monitoring data comprising performing image analysis of an interface of a determining real-time continuous glucose monitoring data application to infer the data from a graph displayed on the interface.

## Description

### FIELD OF THE INVENTION

The present invention relates to devices and methods for monitoring sugar level. In particular, the present invention relates to the use of data acquired from a smart patch like a small water-resistant sensor applied to the skin of a patient, for example on the back of the upper arm.

### BACKGROUND OF THE INVENTION

People suffering from diabetes need to closely and continuously monitor their sugar level during the day.

To this end, several solutions have been provided like finger prick blood tests consisting in taking a drop of blood from the tip of a finger and depositing it on a sugar level measurement device. More recently, solutions a have been developed involving automatic measurements.

With these solutions, the patient is equipped with a small non-invasive patch on the skin that is able to regularly measure sugar level in the blood. Such patches usually use iontophoresis to extract glucose across the skin and to make the measurement. However, other technologies may be used.

The data generated is then stored in view of uploading it wirelessly into a data processing device.

Such data processing device is usually a smartphone with specific software installed on it. The application uses wireless communication capabilities of the smartphone to communicate with the patch. Usually, the communication between the patch and the smartphone is done using, for example, RFID technology (other technologies may be used like Bluetooth® or the like). The user launches the app and puts the smartphone close to the patch. When the smartphone is in a sufficient vicinity to the patch, it is activated to transmit sugar level data stored in using an antenna. The application on the smartphone then processes the data received for several purposes.

The first usual processing of the data received is the transmission thereof to a distant server. This first processing is not really directly useful to the patient and is more useful to the provider of the application, for statistical processing for example.

The second usual processing is the display of a running graph of the sugar level over time (for example the last 8 hours or so). This second processing is more useful to the patient because it makes it possible for her or him to monitor her or his sugar level during the day. The patient can then take several actions like adapting the dose of insulin she or he has to take. The patient can also take other decisions and actions.

However, such applications suffer from several drawbacks and do not offer optimal processing of the data measured by the patch to the patient.

The applications available on the market have poor versatility in terms of display format. The way the data is displayed to the patient is of importance in order to make her or him well understand the data and take the best decision concerning the insulin dose to take. The applications available on the market simply display raw data, as received from the patch, with no scale, no explanation, no direction as to the interpretation, no comparison with standard levels or the like.

In fact, the applications do not really process the data received, except for the direct and raw display of a curve on the smartphone's screen. The computation capabilities of the smartphone are not used. There is no smart use of the data collected.

The absence of any indication of how to interpret the data is a concern for the patient because, at least the error margin of the measurement should be indicated. This margin is available on the instructions manual of the patch but is not readily displayed to the patient. The knowledge of this margin may be useful to the patient to better monitor her or his sugar level. In fact, the absence of knowledge of this margin can be an issue for the patient. Without any data available for comparing the result of the sugar measurement, the patient is left in the dark as to how to interpret her or his current sugar level.

Another severe drawback of the applications available on the market is the absence of interoperability with other applications or devices. The data acquired from the patch is available for raw display on the screen only. The patient cannot make any other use of them, directly or through another application.

Thus, there is a need for improving of the processing of data from smart sugar monitoring devices.

The present invention lies within this context.

### SUMMARY OF THE INVENTION

According to a **first aspect of the invention,** there is provided a computer-implemented method as defined in claim 1.

Embodiments are defined in the dependent claims.

According to a **second aspect of the invention,** there also is provided a device according to perform a method according to the first aspect.

### BRIEF DESCRIPTION OF THE DRAWINGS

The invention will be more clearly understood from the following description, given by way of example only, with reference to the accompanying drawings, in which:
**Figure 1** illustrates a context of implementation of embodiments.
**Figure 2** illustrates interfaces of a Real-time continuous glucose monitoring application.
**Figure 3** illustrates a use case according to embodiments.
**Figure 4** illustrates a use case according to embodiments.
**Figure 5** is a flowchart of steps of a method according to embodiments.
**Figure 6** schematically illustrates a device according to embodiments.

### DETAILED DESCRIPTION OF THE INVENTION

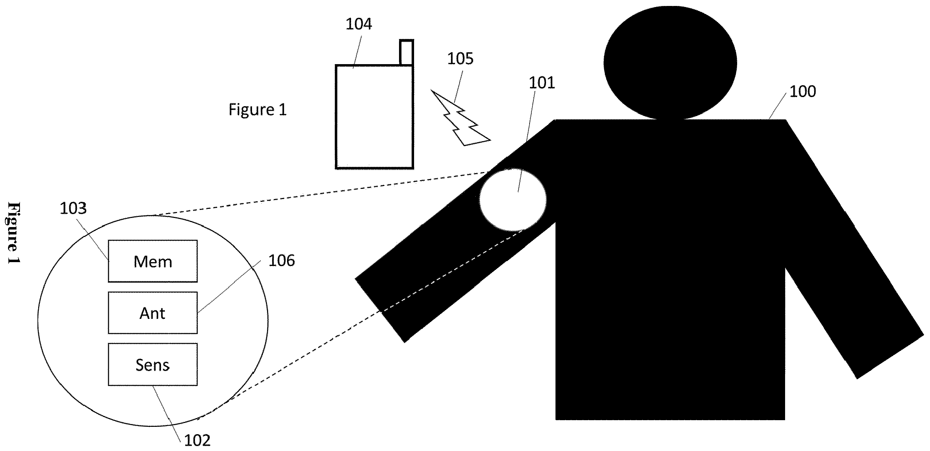

As illustrated in **Figure 1****,** in a general context of implementation of embodiments of the present invention, a patient **100** is provided with a smart glucose measurement patch **101** on the skin. For example, the patch is a non-invasive patch placed on the back of the upper arm.

The patch regularly performs measurements of sugar level using a sensor **102,** for example by iontophoresis and stores the measurement data in a memory **103.**

When the user wants to know her or his current sugar level in the blood, she or he launches an application on a wireless device **104,** like a smartphone. The device is equipped with data processing capabilities for running the application and for performing wireless communications, through a wireless network and also direct wireless communications (Bluthooth®, RFID, or the like). In case RFID technology is uses, the user then places the device **104** close to the patch **101** and a wireless communication **105** is triggered. In case other technologies are used, there may be no need to bring the device in proximity to the patch. The data collected by the sensor **102** and stored in the memory **103** is uploaded into the device through an antenna **106.** The patch **101** may comprise other modules like a control unit (not represented) to control the various parts described above.

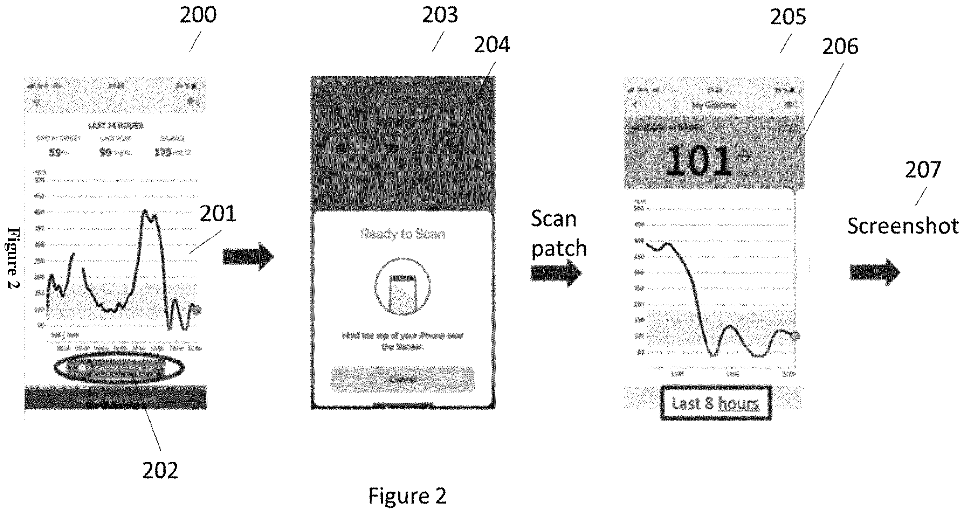

**Figure 2** illustrates the operation of the application of the device 104 by the patient. The interface screenshots shown are purely illustrative.

In a first step **200,** the user launches the application and an interface **201** is displayed showing a curve representing the measurements taken from the patch in the last 24 hours. The Sugar level is given in mg/dL. It could also be given in mmol/l. Other information may be given like for example the last value scanned (in the example of Figure 2: 99 mg/dL) or the average sugar level in the last 24 hours (in the example of Figure 2: 175 mg/dL). Another exemplary data displayed is the time in target which is the percentage of time the patient is within a specified glycemic range (in the example of Figure 2: 59%).

The interface also has a button **202** for launching a sugar level check. When the user hits the button in step **203,** a new interface **204** is displayed. This interface indicates that the application is ready for communication with the patch. In case RFID technology is used, the patient then takes the device close to the patch for downloading data. Otherwise, the data download may start automatically.

Once the data is scanned in step **205,** an interface **206** is displayed showing the last sugar data acquired (in the example of Figure 2: 101 mg/dL) along with the other data from the last measurements (for example from the last 8 hours). Other data can be displayed like the fact that the current data is in a safe range or not. Another type of indication may be the fact that the sugar level is increasing, decreasing or staying stable, using an arrow.

Then, according to embodiments, the user performs a screenshot of the current interface and saves an image representing the data acquired and displayed in a step **207.**

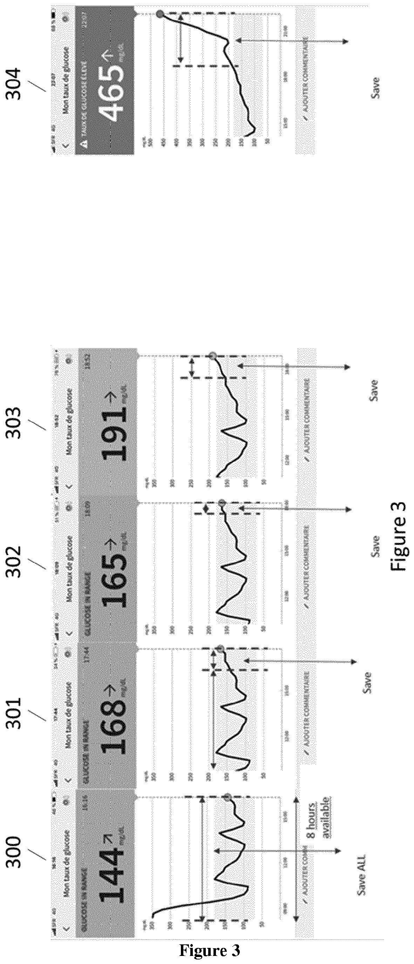

**Figure 3** is an illustration of a use case of embodiments of the invention.

For the sake of the illustration, we consider that screenshot **300** is the first one made by the patient after downloading data from the patch. The interface shows a scan time at 16:16. Since the patch has a memory corresponding to, e.g., 8 hours of measurements, the graph shows the evolution of the sugar level during this time period. As already explained, the interface may shows other data like the current sugar level, the indication of the evolution etc.

According to embodiments, the application performs an analysis of the screen shot to determine the values associated with the graph. In other words, starting from the image data representing the screenshot, the application reconstructs the set of data acquired by the patch (including for example the value of the instant level of sugar, the scale of the graph etc.). The use of image analysis is a solution to the problem users are faced with using applications of the market since, the data are not available. The data are sent to servers of the provider of the patch but are not shared. They are not accessible from the applications of the market and they are not shared with the application developers. With an analysis of the screenshot, the invention makes it possible to get access to the data for further processing.

Since we consider screenshot **300** is the first made by the user and 8 hours of data are available, the application analyses the image and stores all the sugar levels presented on the graph.

In the exemplary use case of **Figure 3****,** the next screenshot **301** made by the user after downloading data from the patch is at 17:44. This screenshot is performed less than 8 hours after the last one. Thus, not all data determined from the image analysis are stored but only data corresponding to sugar levels after 16:16 (last screenshot). The data before 16:16 have already been stored.

The next screenshot **302** is taken at 18:09 after downloading data from the patch. Like for the earlier screenshot, only data that has not already been stored is recorded, i.e. data after 17:44.

The next screenshot **303** is taken at 18:52 after downloading data from the patch. The new data appearing on the screen shot and not already stored are recorded. In this screenshot, the current sugar level is stable (the arrow horizontal) but is now outside the safe range. This is why the indication relating to the fact that the sugar level is in range is absent.

Now, we consider that the patient had dinner and makes a screenshot **304** just after at 22:07 and after downloading data from the patch. The graph shows a big increase of the sugar level which is indicated by an arrow just next to the current sugar level 465 mg/dL. A message is also displayed concerning the fact that the sugar level is high. Just like for the other screenshots, only data acquired after the last screenshot (at 18:52) are saved.

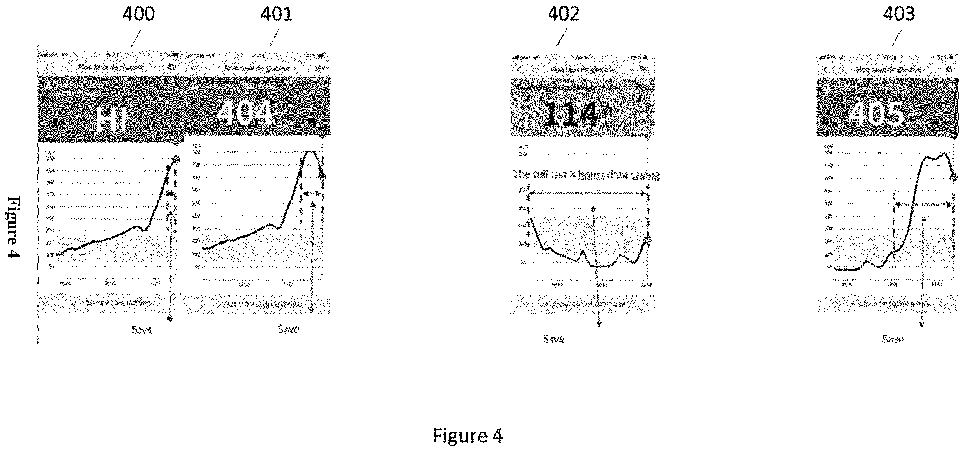

**Figure 4** continues the use case of **Figure 3****.**

The next screenshot **400** is taken at 22:24, soon after the last screenshot and after downloading data from the patch. Only data that has not already been stored are recorded, i.e. data after 22:07. After the sugar level increase noted in screenshot **304,** this level has not decreased and is now out of range. This is indicated to the patient using a dedicated massage in the upper part of the graphical interface. The user then continues the monitoring and makes a new screenshot **401** after downloading data from the patch at 23:14. Only data after 23:14 are stored. The sugar level is now decreasing (as indicated by the arrow but is still high).

The patient then goes to sleep and at 9:03 in the morning after makes a new download of data from the patch and a screenshot **402.** The time between screenshots **401** and **402** is more than 8 hours, therefore, all the data from the graph is stored. The screenshot shows that the sugar level is normal but increasing.

The last screenshot **403** of the use case is performed at 13:06. Data after 9:03 are stored.

In the use case above, each time sugar level data are stored after image data analysis, they may be sent to a distant server for processing (for example statistical processing). The sending may also be performed by batch, for example after a certain number of sugar levels have been determined after image data analysis.

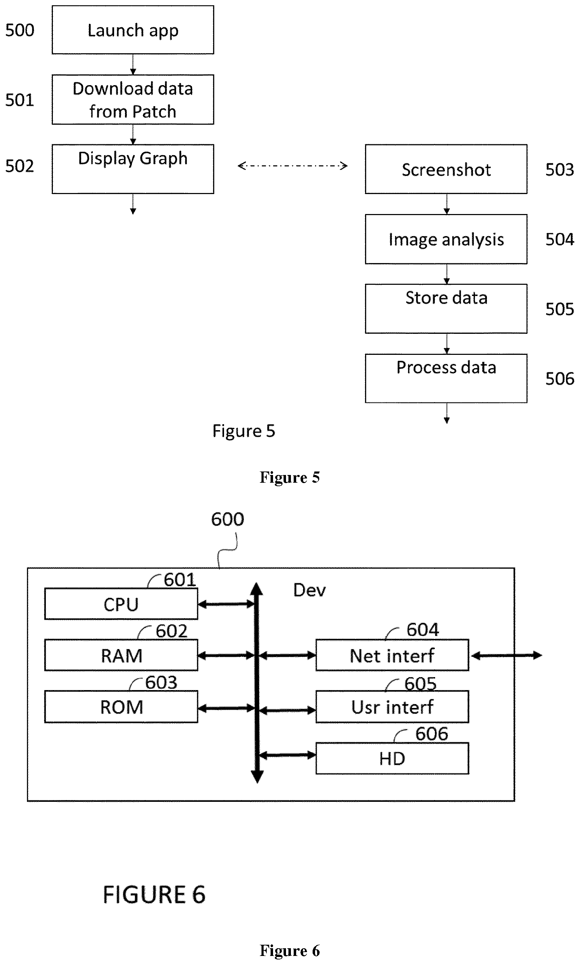

Methods according to embodiments are compatible with any Real-time continuous glucose monitoring applications which display graphs showing the evolution of sugar level with time. As illustrated in **Figure 5****,** such applications can be launched in a step **500** so a user can trigger download of data from a patch in a step **501.** Then, the graph is displayed in a step **502.**

The user can then perform a screenshot of the graph displays in a step **503.** The user can use the capabilities of the device running the Real-time continuous glucose monitoring applications (like a smartphone).

An analysis of the screen shot image it then performed by computer vision technology in step **504** to detect the graph displayed, the corresponding sugar levels, the timing, the numeric scales of the two axis etc. This makes it possible to analyze, process and understand each image acquired, in the current case it would be the screenshot of the blood glucose graph available on the mobile device of the user.

Specifically, pixels from the abscissa and ordinate graph are extracted, processed, classified and categorized into high dimensional data sets from the screen shot in order to produce numerical information that describe the time and blood glucose data by isolating the specific pixels of the blood glucose curve and time. The final rendering of the machine is a digitalized copy of the blood glucose graph evolution in time produced by vision technology software programmed in the mobile device.

Once the sugar level data is derived from the graph, they are stored in a step **505** for further processing in step **506.** The processing may include sending the data to a server for statistical treatment or storing in an account associated with the patient. The processing may also include determination of user specific indications.

The method according to embodiments makes it possible to take advantage of data acquired by the patch but that it not readily available to users. The data can serve a lot of purposes and it is then possible to make different treatments not available in the applications of the market like comparisons, application of algorithm to determine patterns in the sugar level of the user. Thus, the use of the patch is not limited to a simple data display only. Thus, the patient can make a smart use of its proprietary personal data relating to her or his sugar level. The patient can access its personal data and decide to check it and make a smart use of it in order to better monitor her or his sugar level and take appropriate and most relevant decisions. A simple and smart use of data can be implemented using means already available to the patient.

For example, using artificial intelligence on a remote serve, it is possible to provide the user with predictions or advice to avoid certain problems.

Methods according to embodiments also make it possible to enhance the graphical interface offered to the patient by enriching the information displayed. Using the further processings made possible by the image analysis, the interface may be enriched with new type of indications like action suggestions, warnings or the like that can be better adapted to the patient because the raw data has previously been analysed and, for example, been correlated with data of a user profile stored on a distant server. Also, data can be displayed with additional indications relating to the devices used by the patient. For example, the error margin of the measurement can be added to the interface. The error margin information may be determined from an identification of the sensor by the application of the portable device or from the user profile in the distant server.

**Figure 6** is a schematic block diagram of a device **600** for implementing of one or more embodiments of the invention. Device 104 or the smartphone described above may have the same structure.

The device **600** comprises a communication bus connected to:
- a central processing unit **601,** such as a microprocessor, denoted CPU;
- a random access memory **602,** denoted RAM, for storing the executable code of the method of embodiments of the invention as well as the registers adapted to record variables and parameters necessary for implementing a method according to embodiments, the memory capacity thereof can be expanded by an optional RAM connected to an expansion port for example;
- a read only memory **603,** denoted ROM, for storing computer programs for implementing embodiments of the invention;
- a network interface **604** is typically connected to a communication network over which digital data to be processed are transmitted or received. The network interface **604** can be a single network interface, or composed of a set of different network interfaces (for instance wired and wireless interfaces, or different kinds of wired or wireless interfaces). Data are written to the network interface for transmission or are read from the network interface for reception under the control of the software application running in the CPU **601;**
- a graphical user interface **605** for receiving inputs from a user or to display information to a user;
- a hard disk **606** denoted HD
- an I/O module **607** for receiving/sending data from/to external devices such as a video source or display

The executable code may be stored either in read only memory **603,** on the hard disk **606** or on a removable digital medium such as for example a disk. According to a variant, the executable code of the programs can be received by means of a communication network, via the network interface **604,** in order to be stored in one of the storage means of the communication device **600,** such as the hard disk **606,** before being executed.

The central processing unit **601** is adapted to control and direct the execution of the instructions or portions of software code of the program or programs according to embodiments of the invention, which instructions are stored in one of the aforementioned storage means. After powering on, the CPU **601** is capable of executing instructions from main RAM memory **602** relating to a software application after those instructions have been loaded from the program ROM **603** or the hard-disc (HD) **606** for example. Such a software application, when executed by the CPU **601,** causes the steps of a method according to embodiments to be performed.

For ease of understanding and illustrative purposes, the embodiments were described by referring mainly to the drawings. However, drawings should not be considered as restrictive but merely exemplary and illustrative. Various modifications can be made to the embodiments by those skilled in the art without departing from the scope of the disclosure as defined by the claims.

## Claims

1. A computer-implemented method for determining real-time continuous glucose monitoring data comprising the following steps:
- obtaining image data representing a graph of evolution of glucose in a patient's blood over time,
- performing image analysis on the image data obtained to detect data relating to a curve of evolution of said glucose,
- inferring said real-time continuous glucose monitoring data from the detected data relating to said curve, and
- storing the inferred data.

2. The computer-implemented method according to claim 1, wherein said image data is obtained from a screenshot on an interface of a real-time continuous glucose monitoring data application.

3. The computer-implemented method according to claims 1 or 2, further comprising transmitting the inferred data to a distant server.

4. The computer-implemented method according to anyone of claims 1 to 3, further comprising a step of comparing the detected data relating to said curve to previously detected curve data, and wherein only inferred data not present in said previously detected curve data is stored.

5. A device comprising:
- means for storing image data representing a graph of evolution of glucose in a patient's blood over time,
- processing means for performing a method according to any one of the preceding claims,
- means for storing the inferred data.
